# EUROPÄISCHE PATENTSCHRIFT

(11) **EP 2 821 060 B1**
(45) Veröffentlichungstag und Bekanntmachung des Hinweises auf die Patenterteilung: **21.09.2016**
(21) Anmeldenummer: 13174472.4
(22) Anmeldetag: 01.07.2013
(51) Int. Cl.: A61K 8/97, A61K 8/68, A61K 8/55, A61K 8/34, A61K 8/14, A61K 8/67, A61Q 19/00, A61K 8/04

(54) **Kosmetisches Kühlgel**
Cosmetic cooling gel
Gel refroidissant cosmétique

(43) Veröffentlichungstag der Anmeldung: 07.01.2015
(73) Patentinhaber: PM-International AG, 1618 Luxembourg (LU)
(72) Erfinder: Golz-Berner, Karin, MC-9800 Monaco (MC); Meinhardt, Horst, DE-56249 Herschbach (LU); Sorg, Rolf, L-5444 Schengen (LU)
(74) Vertreter: Gulde & Partner

(56) Entgegenhaltungen:
- EP-A2- 1 281 393
- EP-A2- 1 332 772
- WO-A1-01/95728
- WO-A1-2013/041611
- WO-A1-2013/062190
- WO-A2-2009/105740
- CN-A- 103 083 197
- RU-C1- 2 184 528
- DATABASE WPI Section Ch, Week 201347 Thomson Scientific, London, GB; Class B04, AN 2013-J61337 XP002718108, & CN 102 961 551 A (QINGDAO SWAN KNITTING CO LTD) 13. März 2013 (2013-03-13)

## Beschreibung

Die Erfindung betrifft ein kosmetisches Kühlgel auf Basis von Pflanzenextrakten.

Aus der DE 10134607 A1 ist eine kosmetische oder dermatologische Zubereitung mit kühlender Wirkung bekannt, die feste Substanzen mit Schmelzpunkten von 28-40°C und positiver Schmelzenthalpie enthalten. Dabei handelt es sich im wesentlichen um Fettsäureester von C₁₆₋C₂₂-Fettsäuren.

In dem Abstract der RU 2184528 C1 wird ein kosmetisches Kühlgel beschrieben, das Spirulinapulver, Extrakte von Laminaria, Roßkastanie, Arnika und Bambus, Kampfer, Menthol, Avocadoöl, Oreganoöl und Zitronenöl enthält.

Der Erfindung liegt die Aufgabe zugrunde, ein Kühlgel auf pflanzlicher Basis mit einem langanhaltenden Kühleffekt zu entwickeln.

Erfindungsgemäß bereitgestellt wird ein kosmetisches Kühlgel mit Pflanzenextrakten, das ein wäßriges Gel ist und einen einwertigen C2-C4-Alkohol enthält sowie weitere kosmetische Hilfsstoffe und das Gel wenigstens drei Pflanzenextrakte umfaßt, die ausgewählt sind unter den Extrakten von Wermut, Weidenrinde, Schisandrabeeren, Guaranafrüchten und Weihrauchharz.

In einer bevorzugten Ausführungsform der Erfindung ist ein Teil der Pflanzenextrakte sowohl in umhüllenden Trägerstoffen eingeschlossen als auch in freier, nicht eingeschlossener Form enthalten.

Das Kühlgel kann vorteilhaft weitere Pflanzenextrakte in freier Form umfassen, ausgewählt unter den Extrakten von Spitzwegerichblättern, Sanddornbeeren, Lavendelblüten und Gemischen davon. Auch diese Extrakte können zugleich sowohl in umhüllenden Trägerstoffen eingeschlossen sein als auch in freier Form in dem Gel vorliegen.

Das Gel kann zusätzlich 0,01 bis 0,1 Gew.-% Nicotinsäure (Pyridin-3-carbonsäure) enthalten, insbesondere 0,02 bis 0,08 Gew.-%.

Das Gel umfaß 5 bis 13 Gew.% eines C₂-C₄-Alkohols, vorzugsweise 5 - 8 Gew.-%. Bevorzugte Alkohole sind Ethanol oder i-Propanol, insbesondere Ethanol.

Es ist bekannt, daß niedere Alkohole eine gewisse Kühlwirkung auf die Haut ausüben infolge ihres niedrigen Siedepunktes und der entstehenden Verdunstungskälte. Es wurde jedoch gefunden, daß durch die erfindungsgemäßen Pflanzenextrakte die Kühlwirkung des Alkohols wesentlich verlängert wird und zwar in einem Ausmaß, wie dies vorher nicht zu erwarten gewesen war.

Der Gehalt der einzelnen Pflanzenextrakte liegt im Bereich von 0,001 bis 2 Gew.-%, bezogen auf das Gesamtgewicht des Gels.

Dabei ist es vorteilhaft, wenn die Gehalte der Extrakte an Weihrauch, Spitzwegerich, Sanddorn oder Lavendel im Bereich von 0,005 bis 1,0 Gew.-%, insbesondere 0,01 bis 0,05 Gew.-% liegen, die von Guarana, Wermut und Weide im Bereich von 0,005 bis 0,7 Gew.-%, insbesondere 0,01 bis 0,5 Gew.-% und die der Schisandrabeere im Bereich von 0,05 bis 1,2 Gew.-%, insbesondere 0,1 bis 0,8 Gew.-%, jeweils unabhängig voneinander.

In der bevorzugten Ausführungsform der Erfindung liegt das Verhältnis von in umhüllenden Trägerstoffen eingeschlossenen Extrakten und solchen, die nicht eingeschlossen frei im Gel vorliegen, im Bereich von 75 : 25 bis 25 : 75, vorteilhaft 70 : 30 bis 30 : 70 und insbesondere im Bereich 60 : 40 bis 40 : 60 und ist auf das Gewicht bezogen.

Das von Boswellia serrata stammende Weihrauchharz wird schon seit Tausenden von Jahren medizinisch eingesetzt. Dabei wird der Trockenextrakt des Harzes Ölen, Pulvern und Pflastern zugegeben, um rheumatische oder arthritische Erkrankungen, Darmentzündungen und Hautkrankheiten (z. B. Neurodermitis) zu behandeln. Weihrauchharz besteht aus mehr als 80 chemischen Verbindungen, wobei die entzündungswidrige Wirkung vor allem auf den Gehalt an Boswelliasäure zurückgeführt wird. Auch Boswellia papyrifera kann vorteilhaft eingesetzt werden.

In dem erfindungsgemäßen Kühlgel kann Weihrauchextrakt (INCI: Boswellia Serrata Resin Extract) als Trockenextrakt eingesetzt werden. Der Trockenextrakt kann aber auch in eine Pre-Dispersion überführt werden, wie dies auf dem Fachgebiet üblich ist, und dann in das Gel eingebracht werden.

Der Weihrauchextrakt kann auch als Flüssigextrakt eingesetzt werden, z. B. INCI: Butylene Glycol, Boswellia Serrata Gum Extract, der durch selektive Lösungsmittel-extraktion oder Solubilisierung gewonnen wird.

Die Rinde der Weide (Salicis cortex) hat bekanntlich fiebersenkende Wirksamkeit und Wirksamkeit bei Kopfschmerzen und rheumatischen Beschwerden. Allerdings werden die Inhaltsstoffe von Weidenrindenextrakten, vor allem Salicin, erst nach Metabolisierungsprozessen im menschlichen Körper aktiviert und zu Salicylsäure umgewandelt. Äußerliche Anwendungen sind bisher kaum beschrieben worden.

Wermutextrakte von Artemisia absinthium oder A. ponticata werden traditionell zur Appetitanregung und zur Behandlung von Verdauungsbeschwerden eingesetzt, z. B. in Magenbittern. Äußerlich wird Wermut bei Hautflechten, Insektenstichen und schlecht heilenden Wunden in der Volksmedizin verwendet. Bevorzugt sind Extrakte der Gesamtpflanze. Der Begriff "Wermutextrakt" erfaßt auch Trockenprodukte, die aus sprühgetrockneten Wermutweinen hergestellt werden.

Die Extrakte der Schisandrabeere, insbesondere von Schisandra chinensis, die auch als Chinesisches Spaltkörbchen, Chinesische Beerentraube oder Chinesischer Limonenbaum bekannt ist, werden ebenfalls seit Hunderten von Jahren als Heilmittel verwendet. Gemäß der DE 10354667 A1 werden Extrakte von Schisandrabeeren zusammen mit Wolfsbeerenextrakt oder Magnoliablätterextrakt als nahrungsergänzendes Mittel zur Verbesserung des Wohlbefindens eingesetzt, wobei allgemein bekannte Wirkungen aus der chinesischen Medizin bei Diabetis mellitus, Erkältungskrankheiten, Depressionen usw. liegen.

Die eingesetzten Extrakte der Schisandrabeere (INCI: Schizandra Chinensis Fruit Extract) werden aus der Gesamtfrucht gewonnen.

Die südamerikanische Lianenart Guarana (Paullinia cupana) wird insbesondere in Form ihrer pulverisierten Samen in gemahlener Form in Nahrungs- und Nahrungsergänzungsmitteln eingesetzt. Guarana soll auch eine leicht fiebersenkende Wirkung haben. Weiterhin sind Anwendungen bei psychosomatischem Streß auf das Haar (DE 10 2009 043 486 A1) und in Bezug auf die Stärkung der Spannkraft der Haut (DE 10 2008 009 469 A1) bekannt geworden.

Erfindungsgemäß eingesetzt wird Paullinia Cupana Fruit Extract (INCI) in dem Kühlgel.

Der Guaranaextrakt kann auch in Form eines Handelsproduktes (Phytexcell Guarana von Alban Muller) mit der INCI-Bezeichnung Glycerin, Butylene Glycol, Water, Paullinia Cupana Seed Extract verwendet werden oder als Paullinia Cupana Fruit Ectract.

Spitzwegerich ist ebenfalls eine alte Heilpflanze mit im wesentlichen entzündungswidriger und adstringierender Wirksamkeit u. a. durch Verringerung des Auftretens freier Radikale. Ein Handelsprodukt ist beispielsweise Phytessence Plantago von Crodarom. Die INCI-Bezeichnung lautet: Water, Glycerine, Plantago Lanceolata Leaf Extract.

Die Sanddornbeere wird üblicherweise in der Kosmetik für Skincare-Produkte und zur Hautregenerierung eingesetzt. Der INCI-Name lautet: Hippophae Rhamnoides (Seabuckthorn) Berries Extract. Das Produkt ist teilweise auch kombiniert mit Rosmarinblatt-Extrakten zur Stabilisierung.

Extrakte aus Lavendelblüten werden als Duftstoffe, in der pharmazeutischen Industrie z. B. als Diuretikum und auch in der Kosmetik eingesetzt (INCI: Lavandula Angustifolia Flower Extract).

Alle genannten Extrakte von Wermut, Weidenrinde, Schisandrabeeren, Spitzwegerichblättern, Sanddornbeeren, Lavendelblüten, Guaranafrüchten und Weihrauchharz werden als Kaltextrakte bei 18-25°C mittels eines mehrwertigen Alkohols wie Propylenglycol oder Butylenglycol oder Glycerin gewonnen, soweit nichts anderes angegeben.

Die Extrakte in dem erfindungsgemäßen Kühlgel sind teilweise in umhüllenden Trägerstoffen eingeschlossen und werden in Form von Liposomen, Cyclodextrinen, Kollagenmatrices oder Wachs- bzw. Gelatinematrices eingesetzt.

Geeignete Cyclodextrine für die Verkapselung sind z. B. β- oder γ-Cyclodextrin infolge ihrer Größe. Kollagenmatrices können z. B. gemäß EP 0627912 eingesetzt werden.

Liposome sind die bevorzugten Formen für die genannten Extrakte. Liposome sind vollständig geschlossene Lipid-Bilayer-Membranen, die ein wäßriges Volumen eingeschlossen enthalten. Bekannt sind unilamellare Vesikel mit Einzelmembran-Bilayer oder multilamellare Vesikel mit Onion-ähnlichen Strukturen. Letztere zeigen eine Mehrfachmembran-Bilayer, von denen jede Bilayer von der nächsten durch eine wäßrige Schicht getrennt ist.

Für die Bildung der Liposomen geeignete Materialien sind beispielsweise Phospholipide wie Phosphatidylcholin, Phosphatidylinositol, Phosphatidylethanolamin, Phosphatidylserin und Lysolecithine oder Gemische davon.

Die Herstellung von z. B. Liposomen mit Gehalten an Pflanzenextrakten erfolgt üblicherweise derart, daß ein Phospholipid oder -gemisch zusammen mit dem Pflanzenextrakt in Wasser gegeben und mit etwa 400 bis 600 U/min für 5 bis 15 Minuten gerührt wird, je nach Konzentration.

Der Begriff "ein Teil der Pflanzenextrakte ist sowohl in umhüllenden Trägerstoffen eingeschlossen als auch in freier Form enthalten" bedeutet, daß wenigstens zwei der Extrakte in eingeschlossener und in freier Form vorliegen, vorzugsweise wenigstens drei der Extrakte und insbesondere liegen alle Extrakte in eingeschlossener und in freier Form vor. "In freier Form" heißt, daß keine Verkapselungsmaßnahmen für die Extrakte mit Liposomen, Cyclodextrinen oder anderen Matrices vorgenommen wurden.

In einer ersten Ausführungsform der Erfindung enthält das kosmetische Kühlgel die Extrakte von Wermut, Weidenrinde und Weihrauchharz sowohl in umhüllenden Liposomen eingeschlossen als auch in freier Form.

In einer weiteren Ausführungsform der Erfindung enthält das Kühlgel Wermut, Weidenrinde, Weihrauchharz, Schisandrabeeren und Guaranafrucht. Alle Extrakte sind sowohl in umhüllenden Liposomen eingeschlossen als auch in freier Form im Gel enthalten.

In einer weiteren Ausführungsform der Erfindung enthält das Kühlgel Wermut, Weidenrinde, Weihrauchharz, Schisandrabeeren und Guarana. Diese Extrakte liegen vollständig in freier Form vor.

Eine bevorzugte Ausführungsform der Erfindung besteht in einem Kühlgel, das die Extrakte von Wermut, Weihrauchharz, Weidenrinde und Schisandrabeere enthält, vorzugsweise alle eingeschlossen in umhüllenden Trägerstoffen und in freier Form.

Eine weitere bevorzugte Ausführungsform der Erfindung besteht darin, daß das Gel die Extrakte von Wermut, Weihrauchharz, Guaranafrucht und Spitzwegerich enthält. Dabei sind alle Extrakte eingeschlossen in Liposomen und in freier Form vorhanden außer Spitzwegerich, der allein in freier Form vorliegt.

Eine weitere bevorzugte Ausführungsform der Erfindung besteht darin, daß das Gel die Extrakte von Wermut, Weihrauchharz, Guaranafrucht und Sanddornbeeren enthält. Dabei sind alle Extrakte eingeschlossen in Liposomen und in freier Form vorhanden. Zusätzlich kann Nicotinsäure enthalten sein.

Eine weitere bevorzugte Ausführungsform der Erfindung besteht darin, daß das Gel die Extrakte von Lavendelblüten, Weihrauchharz, Weidenrinde, Schisandrabeere und Spitzwegerich enthält. Dabei sind alle Extrakte eingeschlossen in Liposomen und in freier Form vorhanden außer Lavendelblüten, die allein in freier Form vorliegen.

Eine weitere bevorzugte Ausführungsform der Erfindung besteht darin, daß das Gel die Extrakte von Wermut, Weidenrinde, Guaranafrucht und Schisandrabeere enthält. Dabei sind alle Extrakte eingeschlossen in Liposomen und in freier Form vorhanden.

Eine weitere bevorzugte Ausführungsform der Erfindung besteht darin, daß das Gel die Extrakte von Wermut, Weihrauchharz, Guaranafrucht und Schisandrabeeren enthält. Dabei sind alle Extrakte eingeschlossen in Liposomen und in freier Form vorhanden außer Schisandrabeeren, die allein in freier Form vorliegen.

Eine weitere bevorzugte Ausführungsform der Erfindung betrifft ein Kühlgel, das Wermut, Weihrauch, Guaranafrucht und Schisandrabeere als Extrakte jeweils allein in freier Form enthält.

Eine weitere bevorzugte Ausführungsform der Erfindung besteht darin, daß das Gel die Extrakte von Wermut, Weihrauchharz, Guaranafrucht, Sanddorn und Spitzwegerich enthält. Dabei sind die Extrakte von Wermut, Weihrauchharz und Guarana eingeschlossen in Liposomen, und in freier Form liegen Wermut, Guarana, Sanddorn und Spitzwegerich vor.

Eine weitere bevorzugte Ausführungsform der Erfindung besteht darin, daß das Gel die Extrakte von Wermut, Weihrauchharz, Weidenrinde und Schisandra in Liposomen enthält. In freier Form liegen die Extrakte von Weide, Schisandra, Lavendel und Spitzwegerich vor.

Bei dem erfindungsgemäßen Gel handelt es sich um ein wäßriges Gel. Das Gel kann auch Alkohol enthalten. Als Gelbildner sind bevorzugt Carbomer, Xanthangummi, Carrageenan, Guargummi, Agar-Agar, Alginate, Carboxymethylcellulose und Hydroxyethylcellulose. Besonders bevorzugt sind Carbomer und Guargummi.

In einer weiteren Ausführungsform der Erfindung enthält das Kühlgel zusätzlich einen Pflanzenextrakt, ausgewählt aus der Gruppe bestehend aus Arnika (INCI: Arnica Montana Flower Extract), Propolis (INCI: Propolis Extract), Beinwell (INCI: Symphytum Officinale Root Extract), Menthol (INCI: 2-Isopropyl-5-methylcyclohexanol) und Gemischen davon. Die Konzentration dieser Extrakte kann im Bereich von 0,01 bis 0,5 Gew.-% liegen, wobei für Beinwell und Propolis 0,01 bis 0,05 Gew.-% bevorzugt sind. Diese Extrakte können ebenfalls sowohl in umhüllenden Trägerstoffen als auch in freier, nicht eingeschlossener Form vorliegen, vorzugsweise in beiden Formen gleichzeitig wie die anderen oben genannten Pflanzenextrakte.

Das Gel enthält üblicherweise neben den Wirkstoffen, dem Gelbildner, Alkohol und Wasser gegebenenfalls mehrwertige Alkohole wie Glycerin, Propylenglycol, Butylenglycol sowie Duft- und/oder Konservierungsstoffe.

Das Zusammenwirken der erfindungsgemäßen Pflanzenextrakte in einem Gel führt zu einer Kühlwirkung auf der Haut, die deutlich über der Wirkung eines wäßrigen Gels liegt und die darüber hinaus auch eine länger anhaltende Wirkung zeigt. Während die Kühlwirkung eines wäßrigen Gels bereits nach 10 bis 20 Sekunden nachläßt und spätestens nach 30 - 60 Sekunden kaum noch wahrnehmbar ist, hält der Kühleffekt des erfindungsgemäßen Gels mehrere Minuten an, vorzugsweise 3 bis 5 Minuten und ist deutlich stärker als zu erwarten ist.

Probandentests haben gezeigt, daß die Kühlwirkung des erfindungsgemäßen Gels von allen Teilnehmern als signifikant stärker eingeordnet wird, auch im Vergleich mit einem Gel, das 6 Gew.-% Ethylalkohol enthält.

Die Erfindung betrifft auch die Verwendung eines wäßrigen Gels, das neben kosmetischen Hilfsstoffen und einem einwertigen C₂-C₈-Alkohol wenigstens drei Pflanzenextrakte enthält, die ausgewählt sind unter den Extrakten von Wermut, Weidenrinde, Schisandrabeeren, Guaranafrüchten und Weihrauchharz zur Kühlung der Haut von Warmblütlern, insbesondere menschlicher Haut.

Bevorzugt ist eine Verwendung, bei der wenigstens ein Teil der Pflanzenextrakte sowohl in umhüllenden Trägern eingeschlossen ist als auch in freier, nicht eingeschlossener Form enthalten ist.

Die Erfindung soll nachstehend durch Beispiele näher erläutert werden. Alle Prozentangaben sind Gewichtsprozente.

**Beispiel 1a/b Kühlgel Ia und Ib**

| | 1a | 1b |
|---|---|---|
| Wasser | q.s. ad 100 % | q.s. ad 100 % |
| Glycerin | 1,5 - 3,0 | 2 |
| Wermutextrakt | 0,05 - 0,2 | 0,06 |
| Weidenrindenextrakt (Salix Alba Bark Extract) | 0,01 - 0,4 | 0,02 |
| Weihrauchharzextrakt (Boswellia Serrata | | |
| Resin Extract) | 0,02 - 0,05 | 0,02 |
| Lecithin | 1,5 - 5 | 2,5 |
| Carbomer | 0,1 - 0,5 | 0,2 |
| Ethanol | 1 - 7 | 6,0 |
| Konservierungsmittel | 0,3 - 0,5 | 0,3 |

### Herstellung

Ein Teil des Wassers wird mit dem Gelbildner verrührt. Separat werden aus Wasser, Lecithin und den Extrakten die Liposome hergestellt. Hier werden gemäß Beispiel 1b aus
0,25 % Wermutextrakt,
0,03 % Weihrauchharzextrakt und
0,015 % Weidenrindenextrakt

Liposome hergestellt. Die Liposome werden in das Gel bei etwa 400 bis 800 U/min eingerührt. Danach werden unter Rühren die restlichen Anteile gemäß Beispiel 1 b der drei Extrakte in freier Form nacheinander in das Gel eingerührt. Abschließend wird das Gel bei 1000 bis 1500 U/min für maximal 2 Minuten bei 30-40°C homogenisiert.

**Beispiel 2a/2b Kühlgel IIa und IIb**

| | 2a | 2b |
|---|---|---|
| Wasser | q.s. ad 100 % | q.s. ad 100 % |
| Wermutextrakt | 0,1 - 0,7.. | 0,5 |
| Weidenrindenextrakt | 0,05 - 0,3 | 0,1 |
| Weihrauchharzextrakt | 0,02 - 0,07 | 0,03 |
| Schisandrabeerenextrakt (Schizandra Chinensis Fruit Extract) | 0,4 - 0,8 | 0,5 |
| Lecithin | 4,0 - 5,0 | 4,5 |
| Carbomer | 0,3 - 0,5 | 0,4 |
| Ethanol | 5,0 - 6,5 | 6,0 |
| Glycerin | 2,0 - 5,5 | 4,0 |

Es wird wie im Beispiel 1 b verfahren, wobei 0,25 % Schisandrabeerenextrakt in Liposomen eingeschlossen und der Rest später in das Gel zugegeben wird.

**Beispiel 3 Kühlge III**

| | |
|---|---|
| Wasser | q. s. ad 100 % |
| Glycerin | 2,0 |
| Wermutextrakt | 0,7 |
| Weidenrindenextrakt | 0,1 |
| Weihrauchharzextrakt | 0,08 |
| Schisandrabeerenextrakt | 0,1 |
| Guaranafruchtextrakt | 0,1 |
| Ethanol | 5,5 |
| Lecithin | 2,5 |
| Carbomer | 0,2 |

Es wird wie im Beispiel 2b verfahren, wobei 0,04 % Guaranafruchtextrakt in Liposomen eingeschlossen und der Rest später in das Gel zugegeben wird.

### Beispiel 4 Kühlgel IV

Es werden die Bestandteile von Beispiel 3 eingesetzt, wobei der Wermutextrakt 0,45 % beträgt und Lecithin nicht enthalten ist. Alle Extrakte werden in freier Form in das Gel eingebracht und dann wird homogenisiert.

**Beispiel 5 Kühlgel V**

| | |
|---|---|
| Wasser | q. s. ad 100 % |
| Glycerin | 2,5 |
| Wermutextrakt | 0,1 |
| Weihrauchharzextrakt | 1,0 |
| Guaranafruchtextrakt | 0,05 |
| Spitzwegerichblätterextrakt | 0,05 |
| Lecithin | 3,0 |
| Carbomer | 0,2 |
| Ethanol | 6,5 |

Es wird ähnlich wie im Beispiel 3 verfahren, wobei die Gesamtmenge des Spitzwegerichblätterextraktes in freier Form in das Gel zugegeben wird.

**Beispiel 6 Kühlgel VI**

| | |
|---|---|
| Wasser | q. s. ad 100 % |
| Glycerin | 2,0 |
| Wermutextrakt | 0,05 |
| Weihrauchharzextrakt | 0,8 |
| Guaranafruchtextrakt | 0,03 |
| Sanddornbeerenextrakt | 0,01 |
| Ethanol | 6,0 |
| Lecithin | 3,0 |
| Carbomer | 0,3 |
| Nicotinsäure | 0,02 |

Die Arbeitsweise entspricht der von Beispiel 1 b, wobei 0,03 % Wermutextrakt, 0,4 % Weihrauch, 0,02 % Guarana und 0,004 % Sanddornbeerenextrakt in Liposomen eingeschlossen und der Rest später zusammen mit der Nicotinsäure in das Gel zugegeben wird.

**Beispiel 7 Kühlgel VII**

| | |
|---|---|
| Wasser | q. s. ad 100 % |
| Glycerin | 2,5 |
| Weidenrindenextrakt | 0,02 |
| Weihrauchharzextrakt | 0,01 |
| Schisandrabeerenextrakt | 0,01 |
| Spitzwegerichblätterextrakt | 0,01 |
| Lavendelblütenextrakt | 0,05 |
| Lecithin | 2,5 |
| Carbomer | 0,2 |
| Ethanol | 5,5 |

Es wird wie im Beispiel 1 verfahren, wobei die Extrakte von Weidenrinde, Weihrauchharz, Schisandrabeeren und Spitzwegerichblätter zu 50 % in Liposomen eingeschlossen sind und der Rest zusammen mit dem Lavendelblütenextrakt später in das Gel als freie Bestandteile zugegeben wird.

**Beispiel 8 Kühlgel VIII**

| | |
|---|---|
| Wasser | q. s. ad 100 % |
| Glycerin | 2,0 |
| Wermutextrakt | 0,05 |
| Weidenrindenextrakt | 0,05 |
| Schisandrabeerenextrakt | 0,1 |
| Guaranafruchtextrakt | 0,2 |
| Lecithin | 2,5 |
| Carbomer | 0,2 |
| Ethanol | 6,0 |

Es wird wie im Beispiel 1 verfahren, wobei 50 % der Gesamtmenge des jeweiligen Extraktes in Liposomen eingeschlossen und die jeweiligen Reste später in das Gel zugegeben werden.

**Beispiel 9 Kühlgel IX**

| | |
|---|---|
| Wasser | q. s. ad 100 % |
| Glycerin | 1,5 |
| Wermutextrakt | 0,1 |
| Weihrauchharzextrakt | 0,01 |
| Schisandrabeerenextrakt | 0,05 |
| Guaranafruchtextrakt | 0,08 |
| Lecithin | 2,5 |
| Carbomer | 0,2 |
| Ethanol | 6,5 |

Es wird wie im Beispiel 1 verfahren, wobei jeweils 60 % der Gesamtmenge der Extrakte von Wermut, Weihrauchharz und Guaranafrucht in Liposomen eingeschlossen und die jeweiligen restlichen 40 % dieser Extrakte zusammen mit dem Schisandrabeerenextrakt später in das Gel zugegeben werden.

### Beispiel 10 Kühlgel X

Es werden die Bestandteile von Beispiel 9 eingesetzt, wobei der Wermutextrakt 0,2 % beträgt und Lecithin nicht enthalten ist. Alle Extrakte werden in freier Form in das Gel eingebracht, anschließend wird homogenisiert.

**Beispiel 11 Kühlgel XI**

| | |
|---|---|
| Wasser | q. s. ad 100 % |
| Wermutextrakt | 0,03 |
| Weihrauchharzextrakt | 0,08 |
| Guaranafruchtextrakt | 0,5 |
| Spitzwegerichblätterextrakt | 0,08 |
| Sanddornbeerenextrakt | 1,1 |
| Carbomer | 0,2 |
| Lecithin | 2,5 |
| Ethanol | 6,0 |
| Nicotinsäure | 0,04 |

Es wird wie im Beispiel 1 verfahren, wobei 50 % der Gesamtmenge der Extrakte von Wermut und Guaranafrucht in Liposomen eingeschlossen werden, Weihrauch zu 100 % und die jeweiligen restlichen 50 % von Wermut und Guarana zusammen mit dem Sanddornfruchtextrakt, dem Spitzwegerichblätterextrakt und der Nicotinsäure später in das Gel zugegeben werden.

**Beispiel 12 Kühlgel XII**

| | |
|---|---|
| Wasser | q. s. ad 100 % |
| Glycerin | 2,0 |
| Wermutextrakt | 0,05 |
| Weidenrindenextrakt | 0,1 |
| Weihrauchharzextrakt | 0,01 |
| Schisandrabeerenextrakt | 0,5 |
| Spitzwegerichblätterextraktt | 0,5 |
| Lavendelblütenextrakt | 0,1 |
| Lecithin | 2,5 |
| Carbomer | 0,3 |
| Ethanol | 5,0 |

Es wird wie im Beispiel 1 verfahren, wobei 0,06 % Weidenrindenextrakt und 0,28 % Schisandrabeerenextrakt in Liposomen eingeschlossen und die jeweiligen Reste später zusammen mit den Extrakten von Lavendelblüten und Spitzwegerichblättern in freier Form in das Gel zugegeben werden.

### Beispiel 13 Vergleichsbeispiele

Mit einer Gruppe von 20 Probanden und 3 erfahrenen Testern wurde die Kühlwirkung verschiedener Gele untersucht. Es wurden gleichzeitig zwei verschiedene Gele durch einen Tester und den Probanden selbst jeweils auf einen Unterarm in gleicher Menge dünn aufgetragen. Raumtemperatur 22°C, relative Luftfeuchtigkeit 50%.

Die Kühlwirkung wurde nach 10 Sekunden, 30 Sekunden, 1 Minute, 2 Minuten, 3 Minuten, 4 Minuten und 5 Minuten festgestellt nach folgendem Schema
- keine Kühlwirkung
- geringe Kühlwirkung
- mittlere Kühlwirkung
- starke Kühlwirkung

Zwischen den Tests bei jedem Probanden war jeweils eine Pause von 10 Minuten.

**Test 1 a**

| | |
|---|---|
| Ort: | linker Unterarm Innenseite |
| Produkt: | wäßriges Gel aus Wasser, Carbomer, Glycerin (ohne Alkohol und ohne Wirkstoffe) |
| Wirkung: | nach 10 Sekunden - "stark" |
| | nach 30 Sekunden - "mittel" |
| | nach 1 Minute - "keine" |

**Test 1 b**

| | |
|---|---|
| Ort: | rechter Unterarm Innenseite |
| Produkt: | wäßriges Gel aus Wasser, Carbomer, Glycerin und 6 % Ethanol (ohne pflanzliche Wirkstoffe) |
| Wirkung: | nach 10 Sekunden - "stark" |
| | nach 30 Sekunden - "stark" |
| | nach 1 Minute - "mittel" |
| | nach 2 Minuten - "gering" |
| | nach 3 Minuten - "keine" |

**Test 2a**

| | |
|---|---|
| Ort: | linker Unterarm Innenseite |
| Produkt: | gemäß Beispiel 1b |
| Wirkung: | nach 10 Sekunden, 30 Sekunden und 1 Minute - "stark" |
| | nach 2 Minuten - "mittel" |
| | nach 3 Minuten - "mittel" |
| | nach 4 Minuten - "gering" |
| | nach 5 Minuten - "keine" |

**Test 2b**

| | |
|---|---|
| Ort: | rechter Unterarm Innenseite |
| Produkt: | wäßriges Gel aus Wasser, Carbomer, Glycerin und 6 % Ethanol (ohne pflanzliche Wirkstoffe) |
| Wirkung: | nach 10 Sekunden und 30 Sekunden - "stark" |
| | nach 1 Minute - "mittel" |
| | nach 2 Minuten - "gering" |
| | nach 3 Minuten - "keine" |

**Test 3a**

| | |
|---|---|
| Ort: | linker Unterarm Innenseite |
| Produkt: | gemäß Beispiel 2b |
| Wirkung: | nach 10 Sekunden, 30 Sekunden, 1 Minute und 2 Minuten - "start" |
| | nach 3 Minuten - "mittel" |
| | nach 4 Minuten - "mittel" |
| | nach 5 Minuten - "gering" |

**Test 3b**

| | |
|---|---|
| Ort: | rechter Unterarm Innenseite |
| Produkt: | gemäß Beispiel 7 |
| Wirkung: | nach 10 Sekunden, 30 Sekunden und 1 Minute - "stark" |
| | nach 1 Minute, 2 Minuten und 3 Minuten - "mittel" |
| | nach 5 Minuten - "gering" |

**Test 4a**

| | |
|---|---|
| Ort: | linker Unterarm Innenseite |
| Produkt: | gemäß Beispiel 3 |
| Wirkung: | nach 10 Sekunden, 30 Sekunden und 1 Minute - "stark" |
| | nach 2 Minuten und 3 Minuten - "mittel" |
| | nach 4 Minuten - "gering" |
| | nach 5 Minuten - "keine" |

**Test 4b**

| | |
|---|---|
| Ort: | rechter Unterarm Innenseite |
| Produkt: | gemäß Beispiel 4 |
| Wirkung: | nach 10 Sekunden, 30 Sekunden und 1 Minute- "stark" |
| | nach 2 Minuten und 3 Minuten - "mittel" |
| | nach 4 Minuten - "gering" |
| | nach 5 Minuten - "keine" |

**Test 5a**

| | |
|---|---|
| Ort: | linker Unterarm Innenseite |
| Produkt: | gemäß Beispiel 9 |
| Wirkung: | nach 10 Sekunden, 30 Sekunden, 1 Minute und 2 Minuten - "stark" |
| | nach 3 Minuten - "mittel" |
| | nach 4 Minuten - "gering" |
| | nach 5 Minuten - "gering" |

**Test 5b**

| | |
|---|---|
| Ort: | rechter Unterarm Innenseite |
| Produkt: | gemäß Beispiel 12 |
| Wirkung: | nach 10 Sekunden, 30 Sekunden und 1 Minute - "stark" |
| | nach 2 Minuten, 3 Minuten und 4 Minuten - "mittel" |
| | nach 5 Minuten - "gering" |

Die Testergebnisse zeigen, daß der Zusatz von Ethanol und dem Gel bereits eine erhöhte Kühlwirkung erbringt (Test 1a und 1b), diese Wirkung jedoch nach 2 Minuten im wesentlichen verschwunden ist. Dagegen zeigen alle Tests mit den erfindungsgemäßen Extrakten für wenigstens 1 bis 2 Minuten eine stärkere Wirkung als Ethanol allein, und die Wirkung ist in den meisten Fällen bis zum Ende der Meßzeit (5 Minuten) noch deutlich spürbar. Somit ist ein langanhaltender Effekt für das erfindungsgemäße Kühlgel klar zu erkennen.

## Patentansprüche

1. Kosmetisches Kühlgel mit Pflanzenextrakten, **dadurch gekennzeichnet, daß** das Gel ein wäßriges Gel ist, das neben weiteren kosmetischen Hilfsstoffen einen einwertigen C₂-C₄-Alkohol enthält und das Gel wenigstens drei Pflanzenextrakte umfaßt, die ausgewählt sind untern den Extrakten von Wermut, Weidenrinde, Schisandrabeeren, Guaranafrüchten und Weihrauchharz, wobei der Gehalt an einwertigem C₂-C₄-Alkohol im Bereich von 5 bis 13 Gew.-% und der Gehalt der einzelnen Pflanzenextrakte jeweils im Bereich von 0,001 bis 2 Gew.-% liegt, bezogen auf das Gesamtgewicht des Gels

2. Kühlgel nach Anspruch 1, worin weitere Pflanzenextrakte von dem Gel umfaßt werden, ausgewählt unter den Extrakten von Spitzwegerichblättern, Sanddornbeeren, Lavendelblüten und Gemischen davon.

3. Kühlgel nach Anspruch 1 oder 2, worin das Gel weiterhin Nicotinsäure umfaßt.

4. Kühlgel nach Anspruch 1, **dadurch gekennzeichnet, daß** wenigstens ein Teil der Pflanzenextrakte sowohl in umhüllenden Trägern eingeschlossen ist als auch in freier, nicht eingeschlossener Form enthalten ist.

5. Kühlgel nach Anspruch 4, worin das Verhältnis von in umhüllenden Trägern eingeschlossenen und freien, nicht eingeschlossenen Pflanzenextrakten im Bereich von 70 : 30 bis 30 : 70 liegt, vorzugsweise 60 : 40 bis 40 : 60.

6. Kühlgel nach Anspruch 4 oder 5, worin der umhüllende Trägerstoff ein Liposom ist, vorzugsweise ein aus Phospholipiden oder Sphingolipiden gebildetes Liposom.

7. Kühlgel nach einem der Ansprüche 1 bis 6, worin der Gehalt an C₂-C₈-Alkohol im Bereich von 5-8 Gew.-% liegt und der Alkohol Ethanol ist.

8. Kühlgel nach einem der Ansprüche 1 bis 7, worin die Extrakte von Wermut, Weihrauchharz und Weidenrinde in Liposomen eingeschlossen und daneben in freier Form in dem Gel vorliegen.

9. Kühlgel nach einem der Ansprüche 1 bis 7, worin das Gel in Liposomen eingeschlossene Extrakte von Weihrauchharz, Wermut, Schisandrabeeren, Guaranafrucht und Weidenrinde umfaßt und in nicht eingeschlossener Form ebenfalls diese Extrakte.

10. Kühlgel nach einem der Ansprüche 1 bis 7, worin das Gel in Liposomen eingeschlossene Extrakte von Wermut, Weihrauchharz und Guaranafrüchen umfaßt und in nicht eingeschlossener Form Extrakte von Guaranafrüchten, Wermut, Sanddornbeeren und Spitzwegerich.

11. Kühlgel nach einem der Ansprüche 1 bis 7, worin das Gel in Liposomen eingeschlossene Extrakte von Wermut, Weihrauchharz, Weidenrinde und Schisandrabeeren umfaßt und in nicht eingeschlossener Form Extrakte von Weidenrinde, Schisandrabeeren, Lavendelblüten und Spitzwegerich.

12. Kühlgel nach einem der Ansprüche 1 bis 7, worin das Gel in Liposomen eingeschlossene Extrakte von Wermut, Weidenrinde, Schisandrabeere und Weihrauchharz umfaßt und die gleichen Extrakte sowie zusätzlich Lavendelblütenextrakt in nicht eingeschlossener Form.

13. Verwendung eines Kühlgels nach Anspruch 1 zur Kühlung der Haut von Warmblütern, insbesondere menschlicher Haut.

14. Verwendung nach Anspruch 13, bei der wenigstens ein Teil der Pflanzenextrakte sowohl in umhüllenden Trägern eingeschlossen ist als auch in freier, nicht eingeschlossener Form enthalten ist.

## Claims

1. A cosmetic cooling gel having plant extracts, **characterized in that** the gel is an aqueous gel which, in addition to other cosmetic auxiliaries, contains a monovalent C₂-C₄ alcohol and the gel comprises at least three plant extracts which are selected from the extracts of wormwood, willow bark, Schisandra berries, guarana fruits and frankincense resin, wherein the content of monovalent C₂-C₄ alcohol is in the range of 5 to 13% by weight and the content of the individual plant extracts is respectively in the range of 0.001 to 2% by weight, based on the total weight of the gel.

2. The cooling gel according to Claim 1, wherein the gel comprises additional plant extracts which are selected from the extracts of ribwort plantain leaves, sea buckthorn berries, lavender blossoms and mixtures thereof.

3. The cooling gel according to Claim 1 or 2, wherein the gel additionally comprises nicotinic acid.

4. The cooling gel according to Claim 1, **characterized in that** at least a portion of the plant extracts is included in enveloping carriers and is also contained in a free, non-included form.

5. The cooling gel according to Claim 4, wherein the ratio of plant extracts included in enveloping carriers and free, non-included plant extracts is in the range of 70 : 30 to 30 : 70, preferably 60 : 40 to 40 : 60.

6. The cooling gel according to Claim 4 or 5, wherein the enveloping carrier substance is a liposome, preferably a liposome formed from phospholipids or sphingolipids.

7. The cooling gel according to any one of Claims 1 to 6, wherein the content of C₂-C₈ alcohol is in the range of 5-8% by weight and the alcohol is ethanol.

8. The cooling gel according to any one of Claims 1 to 7, wherein the extracts of wormwood, frankincense resin and willow bark are included in liposomes and, in addition, are present in a free form in the gel.

9. The cooling gel according to any one of Claims 1 to 7, wherein the gel comprises extracts of frankincense resin, wormwood, Schisandra berries, guarana fruit and willow bark included in liposomes and also these extracts in a non-included form.

10. The cooling gel according to any one of Claims 1 to 7, wherein the gel comprises extracts of wormwood, frankincense resin and guarana fruits included in liposomes and extracts of guarana fruits, wormwood, sea buckthorn berries and ribwort plantain in a non-included form.

11. The cooling gel according to any one of Claims 1 to 7, wherein the gel comprises extracts of willow bark, wormwood, frankincense resin and Schisandra berries included in liposomes and extracts of willow bark, Schisandra berries, lavender blossoms and ribwort plantain in a non-included form.

12. The cooling gel according to any one of Claims 1 to 7, wherein the gel comprises extracts of wormwood, willow bark, Schisandra berries and frankincense resin included in liposomes and the same extracts as well as, in addition, lavender blossom extract in a non-included form.

13. Use of a cooling gel according to Claim 1 for cooling the skin of warm-blooded animals, in particular human skin.

14. Use according to Claim 13, in which at least a portion of the plant extracts is included both in enveloping carriers and is contained in a free, non-included form.

## Revendications

1. Gel refroidissant cosmétique avec des extraits de plantes, **caractérisé en ce que** le gel est un gel aqueux, lequel comprend, en plus d'autres agents auxiliaires cosmétiques, un mono-alcool C₂-C₄, et **en ce que** le gel comprend au moins trois extraits de plantes, lesquels sont sélectionnés parmi les extraits d'*Artemisia,* d'écorce de saule, de baies de Schisandra, de fruits du guarana et de résine d'arbre à encens, la teneur en mono-alcool C₂-C₄ se trouvant dans la plage de 5 à13 % massique et la teneur des différents extraits de plantes se trouvant respectivement dans la plage de 0,001 à 2 % massique, par rapport au poids total du gel.

2. Gel refroidissant selon la revendication 1, d'autres extraits de plantes étant compris par le gel, sélectionnés parmi les extraits de feuilles de plantain lancéolé, de baies d'argousier, de fleurs de lavande et de mélanges de ceux-ci.

3. Gel refroidissant selon la revendication 1 ou 2, le gel comprenant de l'acide nicotinique.

4. Gel refroidissant selon la revendication 1, **caractérisé en ce qu'**au moins une partie des extraits de plantes est aussi bien enfermée dans des supports enveloppants que contenue sous forme libre, non enfermée.

5. Gel refroidissant selon la revendication 4, le rapport entre les extraits de plantes enfermés dans des supports enveloppants et les extraits de plantes libres, non enfermés se trouvant dans une plage de 70:30 à 30:70, de préférence de 60:40 à 40:60.

6. Gel refroidissant selon la revendication 4 ou 5, la substance porteuse enveloppante étant un liposome, de préférence un liposome formé par des phospholipides ou des sphingolipides.

7. Gel refroidissant selon l'une des revendications 1 à 6, la teneur en alcool C₂-C₈ se trouvant dans la plage de 5 à 8 % massique et l'alcool étant l'éthanol.

8. Gel refroidissant selon l'une des revendications 1 à 7, les extraits *d'Artemisia,* de résine d'arbre à encens et d'écorce de saule existant enfermés dans des liposomes et également sous forme libre.

9. Gel refroidissant selon l'une des revendications 1 à 7, le gel comprenant des extraits de résine d'arbre à encens, *d'Artemisia,* de baies de Schisandra, de fruit du guarana et d'écorce de saule enfermés dans des liposomes et les susdits extraits également sous forme non enfermés.

10. Gel refroidissant selon l'une des revendications 1 à 7, le gel comprenant des extraits d'Artemisia, de résine d'arbre à encens et de fruits du guarana enfermés dans des liposomes et, également, sous forme non enfermés, des extraits de fruits du guarana, *d'Artemisia,* de baies d'argousier et de plantain lancéolé.

11. Gel refroidissant selon l'une des revendications 1 à 7, le gel comprenant des extraits *d'Artemisia,* de résine d'arbre à encens, d'écorce de saule et de baies de Schisandra enfermés dans des liposomes et, sous forme non enfermés, des extraits d'écorce de saule, de baies de Schisandra, de fleurs de lavande et de plantain lancéolé.

12. Gel refroidissant selon l'une des revendications 1 à 7, le gel comprenant des extraits *d'Artemisia,* d'écorce de saule, de baies de Schisandra et de résine d'arbre à encens enfermés dans des liposomes et les mêmes extraits ainsi que, en outre, de l'extrait de fleurs de lavande, sous forme non enfermés.

13. Utilisation d'un gel refroidissant selon la revendication 1 pour le refroidissement de la peau d'animaux à sang chaud, en particulier la peau humaine.

14. Utilisation selon la revendication 13, lors de laquelle au moins une partie des extraits de plantes est aussi bien enfermée dans des supports enveloppants que contenue sous forme libre, non enfermée.
